# EUROPEAN PATENT SPECIFICATION

(11) **EP 4 036 084 B1**
(45) Date of publication and mention of the grant of the patent: **28.01.2026**
(21) Application number: 20868606.3
(22) Date of filing: 23.09.2020
(51) Int. Cl.: C07D 301/12, C07D 301/14, C07B 61/00, C07D 303/04

(54) **METHOD FOR PRODUCING PROPYLENE OXIDE**
VERFAHREN ZUR HERSTELLUNG VON PROPYLENOXID
PROCÉDÉ DE PRODUCTION D'OXYDE DE PROPYLÈNE

(30) Priority: 25.09.2019 JP 2019174095
(43) Date of publication of application: 03.08.2022
(73) Proprietor: Sumitomo Chemical Company, Limited, Tokyo 103-6020 (JP)
(72) Inventor: MUTO Shigeru, Ichihara-shi, Chiba 299-0195 (JP); SARUTA Masaru, Ichihara-shi, Chiba 299-0195 (JP); OONO Kohei, Ichihara-shi, Chiba 299-0195 (JP); IKEDA Shoko, Ichihara-shi, Chiba 299-0195 (JP)
(74) Representative: Vossius & Partner Patentanwälte Rechtsanwälte mbB
(86) International application number: PCT/JP2020/035767
(87) International publication number: WO 2021/060263

(56) References cited:
- JP-A- 2002 173 479
- JP-A- 2002 263 505
- JP-A- 2009 545 554

## Description

### TECHNICAL FIELD

The present invention relates to a method for producing propylene oxide. More specifically, the present invention relates to a method for producing propylene oxide from propylene by epoxidation reaction using a peroxide.

### BACKGROUND ART

A method for continuously producing propylene oxide by supplying a peroxide and propylene to an epoxidation reactor filled with a catalyst then conducting epoxidation reaction is publicly known. JP 2002/263505 A discloses a method for regenerating a solid catalyst used for producing propylene oxide by epoxidizing propylene and an organic peroxide in a reactor packed with a solid catalyst, the method comprising: A method for regenerating a solid catalyst in which a liquid is passed through the catalyst at a temperature higher than the maximum reaction temperature of the epoxidation reaction.

### SUMMARY OF INVENTION

### TECHNICAL PROBLEM

In a case where activity of a catalyst is reduced, for instance, production of propylene oxide is sometimes stopped and a reactor is opened to change the catalyst. In this case, a product and an organic substance such as an unreacted raw material in the reactor need to be removed before opening the reactor. When an inert gas is supplied to the reactor after stopping production of propylene oxide by stopping supply of a peroxide-containing liquid and propylene to the reactor, the organic substance may not be completely removed, and a significant quantity of the organic substance may remain on the catalyst, or long time may be required for sufficiently removing the organic substance.

### SOLUTION TO A PROBLEM

The present invention is as described in the appended claims.

### ADVANTAGEOUS EFFECT OF INVENTION

The present invention allows an organic substance to be efficiently removed when the production of propylene oxide is stopped.

### BRIEF DESCRIPTION OF DRAWING

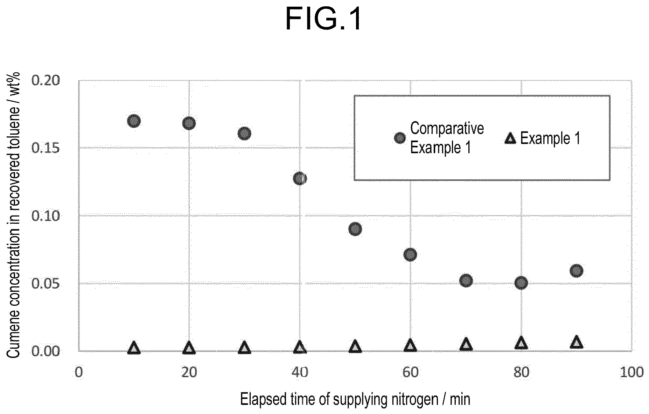

[Fig. 1] Fig. 1 is a graph for confirming residual cumene amounts in Example 1 and Comparative Example 1.

### DESCRIPTION OF EMBODIMENTS

### Definition

In the present specification, a description representing a numerical value range such as "a lower limit to(-) an upper limit" represents "a lower limit or more and an upper limit or less". That is, such a description represents a numerical value range including the lower limit and the upper limit.

### Epoxidation reaction

In the present invention, propylene oxide is produced by supplying a peroxide(-containing liquid) and propylene to an epoxidation reactor, then conducting an epoxidation reaction. The production of propylene oxide is carried out under a pressurized condition.

Examples of the peroxide include hydrogen peroxide and an organic peroxide. The peroxide reacts with propylene to produce propylene oxide and the corresponding hydroxyl compound. The organic peroxide is a compound having the following formula:

R-O-O-H

wherein R is a hydrocarbon group. R in the formula is preferably a C3-20 hydrocarbon group and more preferably a C3-10 hydrocarbon group. The hydrocarbon group may be linear or branched and may be aliphatic or aromatic. Examples of the organic peroxide include cumene hydroperoxide (hereinafter also referred to as CMHP), ethylbenzene hydroperoxide, and tert-butyl hydroperoxide. These may be used singly or a mixture of two or more of these may be used. The organic peroxide supplied to the epoxidation reactor is synthesized by oxidation of a corresponding hydrocarbon compound.

When hydrogen peroxide is used as the peroxide, the corresponding hydroxyl compound is water. When ethylbenzene hydroperoxide is used as the peroxide, the corresponding hydroxyl compound is 1-phenylethanol. When tert-butyl hydroperoxide is used as the peroxide, the corresponding hydroxyl compound is tert-butyl alcohol. When CMHP is used as the peroxide, the corresponding hydroxyl compound is 2-phenyl-2-propanol. Cumene is obtained from this 2-phenyl-2-propanol through dehydration reaction and hydrogenation reaction. Further, CMHP is obtained again by oxidizing the cumene. From these viewpoints, CMHP is preferably used as the organic peroxide needed for epoxidation reaction.

The epoxidation reaction can be conducted in a liquid phase by using a solvent, a diluent, or a mixture thereof. The solvent and the diluent are liquids at a reaction temperature and a reaction pressure. For example, when hydrogen peroxide is used as the peroxide, a liquid containing at least one selected from the group consisting of water, methanol, and acetonitrile can be also used as the solvent and the diluent.

The solvent may consist of a substance existing in a peroxide solution used. For example, in a case where CMHP is mixed with cumene, which is a raw material of CMHP, in a case where ethylbenzene hydroperoxide is mixed with ethylbenzene, which is a raw material of ethylbenzene hydroperoxide, and in a case where tert-butyl hydroperoxide is mixed with isobutane, which is a raw material of tert-butyl hydroperoxide, they can also be substitutes for a solvent without particularly adding a solvent.

An epoxidation reaction temperature is preferably 10 to 200°C, more preferably 25 to 200°C, and further preferably 30 to 200°C. An epoxidation reaction pressure may be a pressure sufficient to keep a reaction phase in a liquid state and is preferably a gauge pressure of 0.1 to 10 MPa and more preferably a gauge pressure of 3 to 9 MPa.

The epoxidation reactor may be a single column or may comprise multiple columns. When multiple columns are comprised, the columns may be connected in series or in parallel. A molar ratio of propylene/(peroxide + the corresponding hydroxyl compound) supplied to the epoxidation reactor is preferably 2/1 to 50/1. If said ratio is less than 2/1, the reaction speed decreases, leading to low efficiency. Meanwhile, if said ratio exceeds 50/1, a large amount of energy is required in processes of separating and recovering an excess amount of unreacted propylene from an epoxidation reaction solution for recycling.

### Catalyst

In the epoxidation reaction in respect of the present invention, a solid catalyst is used for the purpose of obtaining a desired substance at a high yield and/or for the purpose of carrying out the reaction under mild conditions. The catalyst is not particularly limited as long as it known as an epoxidation reaction catalyst. Examples of the catalyst include a titanium-containing silicon oxide catalyst. The titanium-containing silicon oxide refers to a compound in which a part of Si in porous silicate (SiO₂) is substituted with Ti. Said compound has a bond represented by -Si-O-Ti. Examples thereof can include a substance in which a titanium compound is supported by a silica carrier, a substance in which it compounded with a silicon oxide by a coprecipitation method or a sol-gel method, and a zeolite compound containing titanium.

More specific examples can include catalysts described in JP 2004-195379 A, JP 3731384 B2, JP 3797107 B2, and the like; catalysts described in US 2005014960, CN 102311363 B, and the like; Ti-supporting silica described in US 2007260074; Ti-MCM-41 described in US 5783167 and the like; Ti-MCM-48 described in JP 7-300312 A and the like; Ti-HMS described in Nature 368 (1994) p321, CN 101348472 B, CN 101307039 B, CN 101279960 B, CN 102872847 B, CN 103030611 B, and the like; Ti-SBA-15 described in Chemistry of Material 14, 1657-1664, (2002) and the like; TS-1 described in Journal of Catalysis 130, 1-8, (1991) and the like; TS-2 described in Applied Catalysis 58, L1-L4, (1990) and the like; Ti-Beta described in Journal of Catalysis 199, 41-47, (2001) and the like; Ti-ZSM-12 described in Zeolites 15, 236-242, (1995) and the like; TAPS0-5 described in Zeolites 15, 228-235, (1995) and the like; Ti-MOR described in The Journal of Physical Chemistry B 102, 9297-9303 (1998) and the like; Ti-ITQ-7 described in Chemical Communications 761-762, (2000) and the like; Ti-UTD-1 described in Zeolites 15, 519-525, (1995) and the like; and Ti-MWW described in Chemistry Letters, 29, 774-775, 2000 and the like and a precursor thereof (for example, JP 2005-262164 A).

The catalyst can be used in a form of a fixed bed. When the titanium-containing silicon oxide is used as the catalyst, the titanium-containing silicon oxide may be powder or a molded body, preferably a molded body. The reaction herein can be conducted by a semi-continuous method or a continuous method.

Specific examples of the present invention include a method for continuously producing propylene oxide by continuously supplying an organic peroxide(-containing liquid) and propylene to a fixed bed reactor filled with a titanium-containing silicon oxide catalyst for epoxidation to conduct epoxidation reaction.

### Taking out of reaction liquid and purification

After the epoxidation reaction, a liquid mixture containing a desired product can be separated from a catalyst composition. Subsequently, the liquid mixture can be purified by an appropriate method. Examples of the method for purification include distillation, extraction, washing, etc. A solvent and unreacted propylene can be recirculated and used again.

In the present invention, continuous reaction is preferably carried out by using a flow reactor. The flow reactor is a reactor comprising an inlet for continuous supply and an outlet for continuous removal of a content.

Unreacted propylene comprised in a reaction liquid can be separated, recovered, and recycled to the epoxidation reactor. Distillation can be used as a method for separating and recovering unreacted propylene. Conditions under which propylene easily vaporizes are usually employed for distillation. While distillation conditions vary depending on a temperature or composition of the reaction liquid supplied to the distillation step, examples thereof can usually include a gauge pressure of 0 to 5 MPa, preferably a gauge pressure of 0 to 3 MPa, a column top temperature of -50 to 150°C, and a column bottom temperature of 50 to 200°C, preferably a column bottom temperature of 80 to 200°C. Additionally, a method in which multiple distillation columns are used to distill propylene in a stepwise manner may be employed.

Unreacted propylene separated and recovered in this manner can be mixed with propylene newly supplied, and supplied to the epoxidation reactor.

### Completion of reaction

After stopping supply of the peroxide(-containing liquid), liquified gas is supplied to the fixed bed reactor, and a liquified gas is contacted with the catalyst in the fixed bed reactor. The liquified gas is usually supplied to the fixed bed reactor filled with the catalyst in a continuous flow manner. The liquified gas is an organic compound having a boiling point of 40°C or lower under atmospheric pressure. Examples of the liquified gas include propylene, propane, butane, butene, propylene oxide, and pentane, and these may be used singly or a mixture of two or more of these may be used. The liquified gas preferably comprises at least one selected from the group consisting of propylene, propane, butane, and butane, and more preferably comprises propylene, which is raw material of propylene oxide.

When the liquified gas comprises propylene, propylene may be continuously supplied directly after the completion of reaction, or supply may be stopped once and propylene may be supplied again. Additionally, after stopping propylene supply, another kind of the liquified gas may be supplied.

Additionally, the liquified gas may comprise an impurity other than the organic compound having a boiling point of 40°C or lower under atmospheric pressure.

A temperature of the liquified gas, which is supplied after stopping supply of the peroxide(-containing liquid), in the fixed bed reactor is 10 to 100 °C, preferably 20 to 100 °C, and more preferably 30 to 100°C. The liquified gas supplied after stopping supply of the peroxide(-containing liquid) is usually liquid. A part of the liquified gas supplied after stopping supply of the peroxide(-containing liquid) may be gas.

A ratio of (a volume of the liquified gas)/(a volume of the epoxidation reactor) supplied after stopping supply of the peroxide(-containing liquid) is preferably 0.1/1 to 2000/1. The ratio is more preferably 0.5/1 to 500/1 and further preferably 1/1 to 200/1. The volume of the liquified gas is calculated with its density as 544 kg/m³.

In one aspect of the present invention, supply of the liquified gas is stopped after contacting the liquified gas with the catalyst. Stopping of liquified gas supply is carried out before the "stopping of inert gas supply" described later.

An inert gas is supplied to the reactor after stopping supply of the peroxide(-containing liquid). The inert gas may be continuously supplied, or supply may be stopped once and the inert gas may be supplied again thereafter. Examples of the inert gas include helium, neon, argon, nitrogen, water vapor, and carbon dioxide, and a gas comprising one kind thereof or a gas comprising a mixed gas of two or more kinds thereof may be used. Preferably, an object is efficiently achieved by using nitrogen or water vapor. The inert gas may contain oxygen as long as a composition of a gas mixed in the reactor has an oxygen concentration equal to or less than its lower explosive limit.

Supply of the inert gas may be carried out at any timing including timings before, after, and simultaneously with contact of the liquified gas with the catalyst and may be carried out at any timing including timings before, after, and simultaneously with stopping of liquified gas supply. Supply of the inert gas is preferably carried out after stopping of liquified gas supply.

It is preferable to supply of the inert gas until a hydrocarbon concentration contained in the inert gas at the reactor outlet is not detected by a gas detector.

The the method of the present invention is conducted under pressurization, and depressurization is conducted after stopping supply of the peroxide(-containing liquid). For example, a gauge pressure may be decreased from 0.1-10 MPa to 0-0.5 MPa.
Depressurization may be carried out at any timing including timings before, after, and simultaneously with supply of the inert gas. Depressurization is preferably carried out before the supply of the inert gas. Further, depressurization is carried out at after the
liquified gas supply (contact of the liquified gas with the catalyst) and may be carried out at any timing including timings before, after, and simultaneously with stopping of liquified gas supply. Depressurization may be carried out once or two or more times. Depressurization is preferably carried out after stopping liquified gas supply.

Additionally, since timings of inert gas supply and stopping of liquified gas supply are not limited as described above, depressurization (D), inert gas supply (I), and stopping of liquified gas supply (L) may be carried out in any of the following orders:
"D, then I, and then L", "D, then L, and then I", "I, then D, and then L", "I, then L, and then D", "L, then D, and then I", "L, then I, and then D", "D and I at the same time, and then L", "D and L at the same time, and then I", "I and L at the same time, and then D", "D, and then I and L at the same time", "I, and then D and L at the same time", "L, and then D and I at the same time", and "D, I, and L at the same time".

### EXAMPLES

Hereinafter, the present invention will be described with examples.

### Example 1

A metal reactor having an inner diameter of 16 mm was filled with a catalyst (3.5 g of a titanium-containing silicon oxide catalyst prepared in accordance with Example 1 of JP 2004-195379 A), the metal reactor comprising a sheath pipe having an outer diameter of 6 mm and comprising a thermometer inside thereof. The catalyst was hold as a fixed bed having a filling length of 6 cm.

### (A) Epoxidation reaction

Propylene was supplied to a reactor at room temperature in a continuous flow manner at a speed of 0.8 g/min, and heating of the reactor was started at the time when the pressure in the reactor system reached a gauge pressure of 2.4 MPa. A cumene solution containing 3.2% by weight of cumene hydroperoxide was supplied at 2.25 g/min at the time when the pressure reached a gauge pressure of 6 MPa and the internal temperature exceeded 80°C. Thereafter, the pressure in the reactor system was adjusted so as to be a gauge pressure of 6.1 to 6.2 MPa, and the temperature of an electric furnace was adjusted so that the highest position in temperature of the catalyst layer was 100°C. After supply of the cumene solution was started, epoxidation reaction of propylene proceeded to produce propylene oxide. Epoxidation reaction was carried out in a continuous flow manner. A reactor outlet liquid was collected for 15 minutes after a lapse of three hours from the start of supply of the cumene solution. This collected liquid is referred to as "reactor outlet liquid (1)".

### (B) Stopping operation of epoxidation reaction

After collecting reactor outlet liquid (1), supply of the cumene solution containing cumene hydroperoxide was stopped while keeping the setting of the electric furnace constant. Additionally, almost at the same time as the stop, the supplying speed of propylene was changed to 1.64 g/min, and supply was continued for 50 minutes. The supplying speed of propylene was changed to 0.8 g/min, and almost at the same time as the change, heating was stopped. After supplying propylene for 14 minutes, supply of propylene was stopped. The temperature of the catalyst layer at this time was 40°C. Thereafter, the pressure in the system was decreased to a gauge pressure of 0 MPa.

### (C) Remaining organic substance amount evaluation

In order to confirm the remaining organic substance amount, the below measurement was carried out.

Nitrogen was supplied from the inlet of the reactor at 180 ml/min, and organic substances comprised in the gas discharged from the reactor were allowed to be absorbed by a container in which 20 g of toluene with a purity of 99.9% was charged and which was set at the outlet. After a lapse of ten minutes, nitrogen was stopped, and the toluene container at the outlet was exchanged for a new one. This operation was repeated nine times, and nitrogen was supplied for 90 minutes in total. Toluene having absorbed organic substances comprised in the gas was analyzed by gas chromatography to measure the cumene concentration in the recovered toluene. Results are shown in Table 1 and Fig. 1.

Toluene that used to absorb organic substances comprised in the gas did not contain cumene.

The weight of the catalyst taken out after the remaining organic substance amount evaluation (C) was 3.6 g. The catalyst was not wet by visual observation at the time of taking the catalyst out. The cumene solution containing cumene hydroperoxide, which was a raw material liquid, and the reaction liquid did not remain on the catalyst.

Incidentally, the concentration of cumene hydroperoxide contained in reactor outlet liquid (1) was 0.8% by weight. The average temperature of the catalyst layer at the time of sampling was 99°C.

**[Table 1]**

| Cumene concentration in recovered toluene (wt%) | | |
|---|---|---|
| Total N2 flow time (minute) | Example 1 | Comparative Example 1 |
| 10 | 0.00 | 0.17 |
| 20 | 0.00 | 0.17 |
| 30 | 0.00 | 0.16 |
| 40 | 0.00 | 0. 13 |
| 50 | 0.00 | 0.09 |
| 60 | 0.00 | 0.07 |
| 70 | 0.01 | 0.05 |
| 80 | 0.01 | 0.05 |
| 90 | 0.01 | 0.06 |

### Comparative Example 1

### (A) Epoxidation reaction

Epoxidation reaction was conducted in accordance with the method described in

### Example 1.

### (B') Stopping operation of epoxidation reaction

After collecting reactor outlet liquid (1), 50 minutes were allowed to pass in a state where setting of the electric furnace was kept constant after stopping supply of the cumene solution containing cumene hydroperoxide and of propylene. Thereafter, heating was stopped, and the pressure in the system was decreased to a gauge pressure of 0 MPa after confirming that the internal temperature became 40°C or less.

### (C) Remaining organic substance amount evaluation

Toluene having absorbed organic substances comprised in the gas was analyzed by gas chromatography in accordance with the method described in Example 1 to measure the cumene concentration in the recovered toluene. Results are shown in Table 1 and Fig. 1.

The weight of the catalyst taken out after the remaining organic substance amount evaluation (C) was 5.8 g. The catalyst was wet by visual observation at the time of taking the catalyst out. The cumene solution containing cumene hydroperoxide, which was a raw material liquid, and the reaction liquid remained on the catalyst.

In the working example, it has been confirmed that the outflow amount of cumene is small and the organic substance can be efficiently removed, compared to the comparative example.

Incidentally, the concentration of cumene hydroperoxide contained in reactor outlet liquid (1) was 0.7% by weight. The average temperature of the catalyst layer at the time of sampling was 99°C.

### INDUSTRIAL APPLICABILITY

The present invention is useful for producing propylene oxide.

## Claims

1. A method for producing propylene oxide, comprising:
supplying a peroxide(-containing liquid) and propylene to a fixed bed reactor filled with a catalyst to produce propylene oxide;
stopping the supplying of the peroxide(-containing liquid);
contacting a liquefied gas with the catalyst in the fixed bed reactor after the stopping of the supplying of the peroxide(-containing liquid); and
supplying an inert gas to the fixed bed reactor,
wherein the production of propylene oxide is carried out under a pressurized condition, and the method comprises depressurizing an inside of the fixed bed reactor after the contact, and
wherein a temperature of the liquified gas in the fixed-bed reactor is 10 to 100 °C.

2. The method according to claim 1, wherein the liquefied gas comprises at least one selected from the group consisting of propylene, propane, butane, butene, propylene oxide, and pentane.

3. The method according to claim 1 or 2, wherein the supplying of the inert gas is carried out before, after, or simultaneously with the contacting of the liquefied gas with the catalyst in the fixed bed reactor.

4. The method according to any one of claims 1 to 3, wherein the production of propylene oxide is carried out at a gauge pressure of 0.1-10 MPa, and the depressurizing is to decrease the gauge pressure from 0.1-10 MPa to 0-0.5 MPa.

5. The method according to any one of claims 1 to 4, wherein the depressurizing is carried out before, after, or simultaneously with the supplying of the inert gas.

6. The method according to any one of claims 1 to 5, wherein the depressurizing is carried out before, after, or simultaneously with the contacting of the liquefied gas with the catalyst in the fixed bed reactor.

7. The method according to any one of claims 1 to 6, wherein the catalyst is a titanium-containing silicon oxide.

8. The method according to any one of claims 1 to 7, wherein the inert gas comprises at least one selected from the group consisting of helium, neon, argon, nitrogen, water vapor, and carbon dioxide.

9. The method according to any one of claims 1 to 8, wherein the peroxide is at least one selected from the group consisting of an organic peroxide and hydrogen peroxide.

10. The method according to any one of claims 1 to 9, wherein the peroxide is at least one selected from the group consisting of cumene hydroperoxide, ethylbenzene hydroperoxide, tert-butyl hydroperoxide, and hydrogen peroxide.

## Patentansprüche

1. Ein Verfahren zur Herstellung von Propylenoxid, umfassend:
Zufuhr eines Peroxids (einer peroxidhaltigen Flüssigkeit) und von Propylen zu einem mit einem Katalysator gefüllten Festbettreaktor, um Propylenoxid herzustellen;
Beenden der Zufuhr des Peroxids (der peroxidhaltigen Flüssigkeit);
Kontaktieren eines Flüssiggases mit dem Katalysator in dem Festbettreaktor nach dem Beenden der Zufuhr des Peroxids (der peroxidhaltigen Flüssigkeit); und
Zufuhr eines Inertgases zu dem Festbettreaktor,
wobei die Herstellung von Propylenoxid unter einer Druckbedingung durchgeführt wird und das Verfahren Druckentlasten eines Inneren des Festbettreaktors nach dem Kontakt umfasst, und
wobei eine Temperatur des Flüssiggases in dem Festbettreaktor 10 bis 100 °C beträgt.

2. Das Verfahren nach Anspruch 1, wobei das Flüssiggas mindestens eines, ausgewählt aus der Gruppe bestehend aus Propylen, Propan, Butan, Buten, Propylenoxid und Pentan, umfasst.

3. Das Verfahren nach Anspruch 1 oder 2, wobei die Zufuhr des Inertgases vor, nach oder gleichzeitig mit dem Kontaktieren des Flüssiggases mit dem Katalysator in dem Festbettreaktor durchgeführt wird.

4. Das Verfahren nach einem der Ansprüche 1 bis 3, wobei die Herstellung von Propylenoxid bei einem Manometerdruck von 0,1-10 MPa durchgeführt wird und die Druckentlastung dazu dient, den Manometerdruck von 0,1-10 MPa auf 0-0,5 MPa zu verringern.

5. Das Verfahren nach einem der Ansprüche 1 bis 4, wobei die Druckentlastung vor, nach oder gleichzeitig mit der Zufuhr des Inertgases durchgeführt wird.

6. Das Verfahren nach einem der Ansprüche 1 bis 5, wobei die Druckentlastung vor, nach oder gleichzeitig mit dem Kontaktieren des Flüssiggases mit dem Katalysator in dem Festbettreaktor durchgeführt wird.

7. Das Verfahren nach einem der Ansprüche 1 bis 6, wobei der Katalysator ein titanhaltiges Siliciumoxid ist.

8. Das Verfahren nach einem der Ansprüche 1 bis 7, wobei das Inertgas mindestens eines, ausgewählt aus der Gruppe bestehend aus Helium, Neon, Argon, Stickstoff, Wasserdampf und Kohlendioxid, umfasst.

9. Das Verfahren nach einem der Ansprüche 1 bis 8, wobei das Peroxid mindestens eines, ausgewählt aus der Gruppe bestehend aus einem organischen Peroxid und Wasserstoffperoxid, ist.

10. Das Verfahren nach einem der Ansprüche 1 bis 9, wobei das Peroxid mindestens eines, ausgewählt aus der Gruppe bestehend aus Cumolhydroperoxid, Ethylbenzolhydroperoxid, tert-Butylhydroperoxid und Wasserstoffperoxid, ist.

## Revendications

1. Méthode pour produire de l'oxyde de propylène, comprenant :
la fourniture d'un (liquide contenant du) peroxyde et de propylène à un réacteur à lit fixe garni d'un catalyseur pour produire de l'oxyde de propylène ;
l'arrêt de la fourniture du (liquide contenant du) peroxyde ;
la mise en contact d'un gaz liquéfié avec le catalyseur dans le réacteur à lit fixe après l'arrêt de la fourniture du (liquide contenant du) peroxyde ; et
la fourniture d'un gaz inerte au réacteur à lit fixe,
dans laquelle la production d'oxyde de propylène est effectuée dans une condition pressurisée, et la méthode comprend la dépressurisation d'un intérieur du réacteur à lit fixe après le contact, et
dans laquelle la température du gaz liquéfié dans le réacteur à lit fixe est de 10 à 100 °C.

2. Méthode selon la revendication 1, dans laquelle le gaz liquéfié comprend au moins l'un choisi dans le groupe constitué par le propylène, le propane, le butane, le butène, l'oxyde de propylène, et le pentane.

3. Méthode selon la revendication 1 ou 2, dans laquelle la fourniture du gaz inerte est effectuée avant, après, ou en même temps que la mise en contact du gaz liquéfié avec le catalyseur dans le réacteur à lit fixe.

4. Méthode selon l'une quelconque des revendications 1 à 3, dans laquelle la production d'oxyde de propylène est effectuée sous une pression manométrique de 0,1-10 MPa, et la dépressurisation consiste à diminuer la pression manométrique de 0,1-10 MPa à 0-0,5 MPa.

5. Méthode selon l'une quelconque des revendications 1 à 4, dans laquelle la dépressurisation est effectuée avant, après, ou en même temps que la fourniture du gaz inerte.

6. Méthode selon l'une quelconque des revendications 1 à 5, dans laquelle la dépressurisation est effectuée avant, après, ou en même temps que la mise en contact du gaz liquéfié avec le catalyseur dans le réacteur à lit fixe.

7. Méthode selon l'une quelconque des revendications 1 à 6, dans laquelle le catalyseur est un oxyde de silicium contenant du titane.

8. Méthode selon l'une quelconque des revendications 1 à 7, dans laquelle le gaz inerte comprend au moins l'un choisi dans le groupe constitué par l'hélium, le néon, l'argon, l'azote, la vapeur d'eau, et le dioxyde de carbone.

9. Méthode selon l'une quelconque des revendications 1 à 8, dans laquelle le peroxyde est au moins l'un choisi dans le groupe constitué par un peroxyde organique et le peroxyde d'hydrogène.

10. Méthode selon l'une quelconque des revendications 1 à 9, dans laquelle le peroxyde est au moins l'un choisi dans le groupe constitué par l'hydroperoxyde de cumène, l'hydroperoxyde d'éthylbenzène, l'hydroperoxyde de tert-butyle, et le peroxyde d'hydrogène.
